Europäisches Patentamt

(19) European Patent Office (11) Numéro de publication: **0 209 536**

Office européen des brevets **B1**

(12) **FASCICULE DE BREVET EUROPEEN**

(45) Date de publication du fascicule du brevet: **25.04.90** (51) Int. Cl.⁵: **A 01 H 5/00**

(21) Numéro de dépôt: **86900173.5**

(22) Date de dépôt: **31.12.85**

(86) Numéro de dépôt international:
**PCT/FR85/00379**

(87) Numéro de publication internationale:
**WO 86/03934 17.07.86 Gazette 86/17**

(54) **PROCEDE DE MULTIPLICATION ET DE CULTURE DES ESPECES BAMBUSIFORMES ET LIGNEUSES, DE LEURS HYBRIDES ET DE LEURS MUTANTS, DU GENRE BOTANIQUE BEGONIA.**

(30) Priorité: **07.01.85 FR 8500123**

(43) Date de publication de la demande:
**28.01.87 Bulletin 87/05**

(45) Mention de la délivrance du brevet:
**25.04.90 Bulletin 90/17**

(84) Etats contractants désignés:
**AT BE CH DE FR GB IT LI LU NL SE**

(56) Documents cités:
**BE-A- 644 953**
**FR-A-1 256 910**
**FR-A-2 293 140**
**FR-A-2 388 485**
**FR-A-2 482 414**
**US-A- 3 750**

**G. Krüsmann: "Die Baumschule", 4ème édition
1978, publié par "Verlag Paul Parey", Berlin (DE),
pages 153-156, 165-167**

(73) Titulaire: **CHAINTRON, Jean-Marc
5, rue Béconcelles
F-78910 Orgerus (FR)**

(72) Inventeur: **CHAINTRON, Jean-Marc
5, rue Béconcelles
F-78910 Orgerus (FR)**

(74) Mandataire: **Warcoin, Jacques et al
Cabinet Régimbeau 26, avenue Kléber
F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 209 536 B1

## Description

La présente invention concerne un procédé de multiplication et de culture des espèces bambusiformes et des espèces ligneuses, de leurs hybrides et de leurs mutants, du genre botanique bégonia.

Ce procédé permet:

d'obtenir des plantes de formes nouvelles pour les espèces considérées,

d'obtenir des plantes de hauteurs standardisées,

d'obtenir des plantes qui fleurissent plus souvent et plus tôt et plus abondamment,

de faire des économies d'énergie, plus de 50 % de la culture pouvant être faite dans les contrées chaudes,

de réduire les coûts de distribution, transport aisé (application facile à la vente par correspondance).

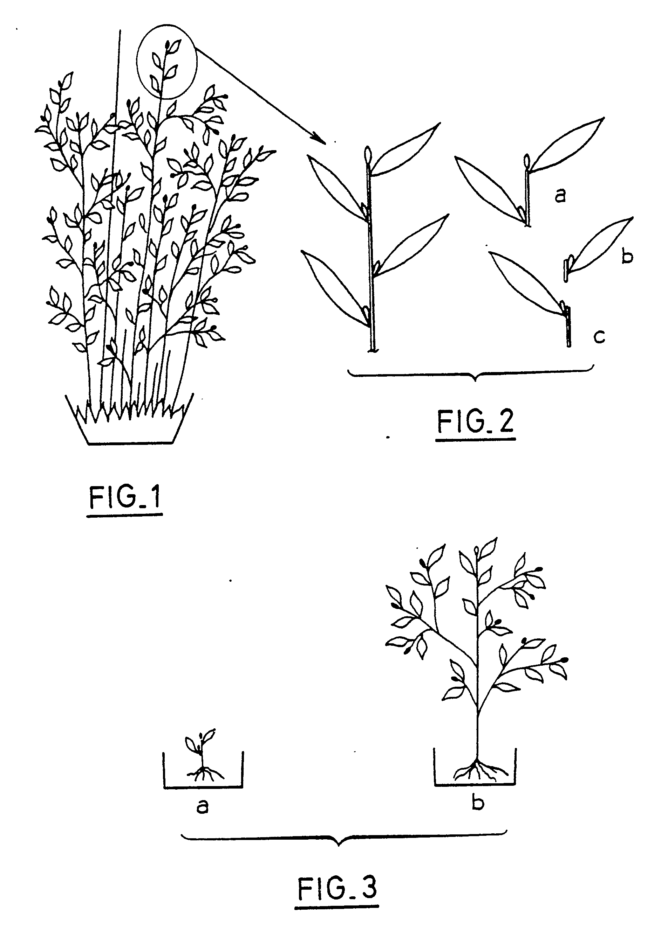

Espèces souvent originaires de la Cordillière des Andes, les espèces bambusiformes, ligneuses, buissonnantes et/ou leurs hybrides, peuvent atteindre des tailles surprenantes (2 à 3 mètres de haut) (figure 1).

Les espèces de bégonias bambusiformes et bégonias ligneux sont multipliées par bouture tout comme beaucoup d'espèces dont la multiplication sexuée est difficile ou de faible rendement.

Les boutures sont faites à partir de parties végétales herbacées, longues de 5 à 10 cm, communément appelées: boutures herbacées de tête (figure 2a), boutures à un (figure 2b) ou deux (figure 2c) entre noeuds. Ces boutures sont de section allant de 5 à 6 mm. Dans les conditions habituelles de culture, tout homme de l'art multiplie ainsi ces plantes.

Après enracinement à la base, la jeune plante ainsi obtenue peut être rempotée en pot ou en pleine terre (figures 3a et 3b). Les ramifications de cette plante et sa végétation sur quelques mois l'amènent à une taille qui va de 20 à 50 cm maximum. Les rameaux portés par la bouture initiale ne permettent pas d'obtenir rapidement une plante de la taille initiale (2 m).

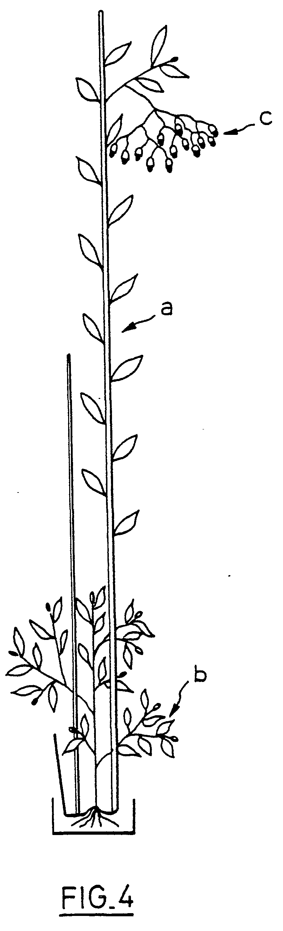

Ceci ne peut se faire (figure 4) qu'après apparition de drageons (a) à la base de la plante (b), ce qui demande sous nos climats près d'un an de culture. Ces drageons donnent des cannes qui, selon les conditions de culture, peuvent atteindre plus de 3 m de haut.

La floraison n'intervient qu'après lignification du bois, d'abord sur les petits rameaux formés en aval de la bouture initiale (figure 4c).

La floraison n'intervient sur les cannes que lorsqu'elles ont atteint une certaine maturité (4 à 6 mois de vie).

Très peu cultivées, voire abandonnées, les plantes hautes, fleuries, sont très longues à produire, coûteuses en énergie sous nos climats, même en serre.

La présente invention concerne un procédé de multiplication et de culture des espèces bambusiformes et des espèces ligneuses, de leurshybrides et de leurs mutants, du genre botanique Bégonia, caractérisé en ce qu'il consiste à prendre comme matériel de multiplication des morceaux de cannes lignifiées et matures avec bourgeons axilaires.

Le procédé de la présente invention consiste à prendre les tiges lignifiées et de section forte communément appelées cannes comme matériel végétal de multiplication et de reproduction, au lieu des parties sarmenteuses et de faible section usuellement employées.

Dès lignification et maturation des cannes, celles-ci sont coupées, débarrassées de leurs feuilles, tronçonnées en morceaux pouvant aller de quelques centimètres à trois mètres de long suivant la taille désirée de la plante ultérieure. Le tronçonnement se fait toujours entre deux noeuds. A partir du stade de lignification qui correspond à l'apparition des premières fleurs sur les cannes, la canne a généralement la maturation désirée.

A partir de cet état, le choix de l'âge auquel les cannes sont cueillies conditionne la forme ultérieure de la plante. Par exemple, une canne très âgée convient mieux pour faire un arbre tige, une canne jeune convient mieux pour créer une colonne. En effet, sur une canne âgée, il est difficile de réveiller la dormance des méristèmes des bourgeons axilaires, quand encore ils sont existants.

Les cannes ou tronçons de cannes obtenus comme décrit ci-dessus peuvent être stockés plusieurs mois dans les conditions habituelles de stockage de fruits et de végétaux (par exemple en chambre froide à 4°C) sans altérer la reprise. Les cannes peuvent facilement être colisées et transportées.

A une date pouvant être espacée de plusieurs mois depuis la récolte, les cannes peuvent être plantées pour s'enraciner. Pour cela, on rafraichit les coupes, c'est-à-dire que l'on coupe la partie supérieure de la canne au ras d'un oeil (bourgeon) et légèrement en oblique.

La partie basse est coupée à 1 cm du noeud. Les coupes doivent être franches et faites de manière à ne pas écraser la canne.

Sur la partie des cannes qui sera enterrée, on excise les bourgeons ou méristèmes dormants au ras du bois de manière à éliminer totalement l'assise génératrice qui produirait des bourgeons, lesquels donneraient des tiges rhizomateuses.

On élimine ainsi toute possibilité de retour à l'aspect buissonnant et on conserve à coup sûr la forme arborescente.

Après rafraichissement des coupes, on plante la canne dans le sol à une profondeur d'au moins 5 cm. Les cannes doivent être plantées dans le bon sens. L'oeil doit être situé au-dessus et dans le sens vertical de la cicatrice du pétiole, qui a été détaché à la récolte.

L'enracinement de ces cannes se fait sans difficulté. Pour certaines espèces, le trempage de la partie qui est enterrée dans des hormones végétales d'enracinement, par exemple, choisies parmi les auxines et les cytokinines ou leurs mélanges, accélère la reprise. Afin de favoriser l'enracinement de la canne et un bon ancrage dans le sol, on enlève le tissu cortical, longitudinalement sur une hauteur de 3 à 4 cm et une largeur de 3 à 7 mm en deux ou trois points répartis judicieusement à équidistance.

Ce procédé permet l'enracinement en surface et la tenue de la canne par les racines de surface sans empêcher l'ancrage et l'enracinement naturel et plus profond situé au niveau de la coupe de la canne.

La taille de la plante obtenue par le mode de multiplication décrit ci-dessus est déterminée au départ par la taille de la canne. Celle-ci est un tronçon d'arbre, sans méristème apical, lignifié et de ce fait elle ne peut plus grandir. La canne ou tronçon de canne initiale, une fois récoltée puis replantée comme ci-dessus décrit, gardera sa taille initiale. Seuls les bourgeons axilaires donneront à la plante une taille ou forme nouvelle. Cependant, les bourgeons axilaires provenant d'une canne mature ne donneront jamais naissance à une nouvelle canne dans la partie supérieure de la plante. Ces bourgeons axilaires donnent toujours naissance à des tiges fines, sarmenteuses (bois secondaire) nombreuses et dont le port est souvent légèrement pleureur.

Ces bois secondaires sont fructifères et porteurs de fleurs en abondance.

Le procédé ci-dessus décrit permet la standardisation des plantes, par exemple si toutes les cannes sont coupées à 50 cm, toutes les plantes qui en découleront auront la même taille dans des conditions de culture identiques.

La floraison des plantes obtenues par le procédé de l'invention est hâtive. La canne confère aux bourgeons axilaires une partie de sa maturation, évitant une rejuvénilisation. Or la maturation des tissus primaires d'une plante confère aux tissus secondaires situés en aval une plus grande aptitude à la floraison, phénomène bien connu par les physiologistes.

Les bourgeons axilaires donnent naissance à des ramifications secondaires dont la taille et les pincements mécaniques (coupe du bout des tiges) ou chimiques ("off shoot") permettent d'obtenir toutes sortes de formes pour une taille donnée de la canne au départ. On peut également obtenir des formes plates, palmettes, cônes inversés, cascades, par exemple.

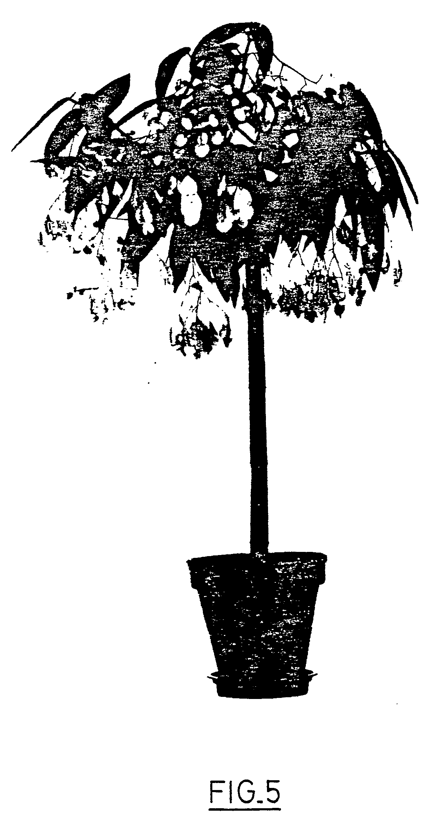

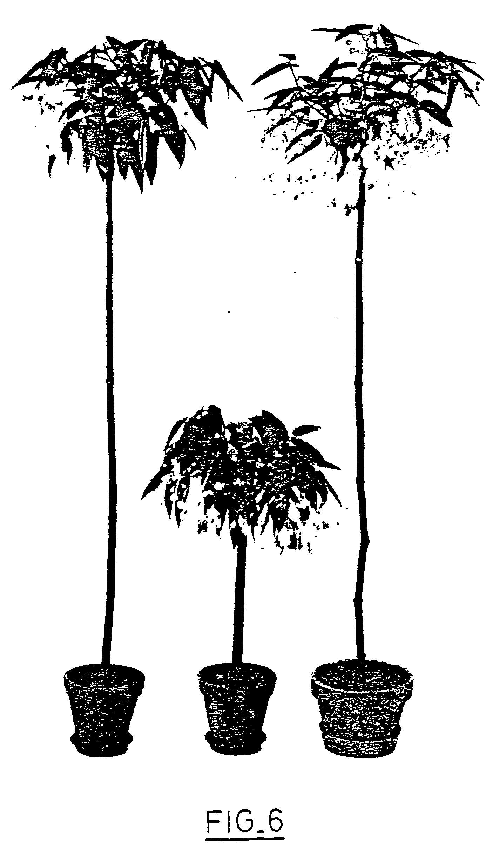

A titre d'exemple, les figures 5 et 6 montrent l'aspect de plantes correspondant à une canne d'environ 50 cm (figure 5) et à des cannes de 1,2 m et 50 cmn (figure 6).

La canne objet du présent brevet peut servir de porte-greffe pour tout genre botanique bégonia couvrant toutes les espèces et variétés des bégoniacées.

Le greffage en écusson, en fente, en biseau, par approche, en tête, etc. permet d'établir et de parfaire la forme des plantes. Pour cela, on greffe sur le tronçon de canne choisi un écusson, c'est-à-dire un bourgeon, que l'on applique sur la canne préalablement préparée.

La plante obtenue par le procédé décrit est très résistante à la sécheresse, au froid, jusqu'à 1°C, au chaud, jusqu'à 45°C, et elle convient bien à la décoration des appartements modernes et des vérandas.

Le procédé de multiplication reproduit à l'identique la variété ainsi multipliée. Il permet de ce fait de satisfaire les besoins de l'industrie horticole.

**Revendications**

1. Procédé de multiplication et de culture des espèces bambusiformes et des espèces ligneuses, de leurs hybrides et de leurs mutants, du genre botanique Bégonia, caractérisé en ce qu'il consiste à prendre comme matériel de multiplication des morceaux de cannes lignifiées et matures avec bourgeons axilaires.

2. Procédé selon la revendication 1, caractérisé en ce que dès lignification et maturation, les cannes sont coupées, débarrassées de leurs feuilles et tronçonnées en morceaux dont la longueur correspond à la taille de la plante désirée.

3. Procédé selon la revendication 2, caractérisé en ce que la coupe initiale des cannes est effectuée entre deux noeuds.

4. Procédé selon l'une des revendications 1 à 3, caractérisé en ce que les cannes sont mises en terre après tronçonnage et stockage éventuel, après rafraîchissement de la coupe au voisinage du noeud.

5. Procédé selon la revendication 4, caractérisé en ce que la partie destinée à être mise en terre est préalablement mise en contact avec des hormones végétales d'enracinement.

6. Procédé selon la revendication 5, caractérisé en ce que les hormones d'enracinement sont choisies parmi les auxines et les cytokinines et leurs mélanges.

7. Procédé selon l'une des revendications 1 à 5, caractérisé en ce qu'on enlève le tissu cortical longitudinalement sur la partie destinée à être mise en terre pour favoriser l'enracinement en surface.

8. Procédé selon l'une des revendications 1 à 7, caractérisé en ce que sur la partie destinée à être mise en terre on excise les bourgeons ou méristèmes dormants au ras du bois.

9. Procédé selon l'une des revendications 1 à 7, caractérisé en ce qu'après mise en terre, la plante est traitée par un produit empêchant le drageonnage.

10. Procédé selon l'une des revendications 1 à 9, caractérisé en ce qu'on effectue, après l'enracinement, une greffe des tronçons de cannes avec une autre variété de Bégoniacées.

**Patentansprüche**

1. Verfahren zur Vermehrung und Kultivation von bambus- und holzartigen Sorten, von deren Hybriden und deren Mutanten des botanischen Genus Begonia, dadurch gekennzeichnet, daß es darin besteht, als Vermehrungsmaterial Stücke von verholzten und ausgereiften Stöcken mit axialen Knospen zu nehmen.

2. Verfahren nach Anspruch 1, dadurch gekennzeichnet, daß von der Verhoizung und der Ausreifung die Stöcke abgeschnitten, von ihren Blättern befreit und in Stücke geschnitten werden, deren Länge der Größe der gewünschten Pflanze entspricht.

3. Verfahren nach Anspruch 2, dadurch gekennzeichnet, daß der erste Schnitt der Stöcke zwischen zwei Knoten ausgeführt wird.

4. Verfahren nach einem der Ansprüche 1 bis 3, dadurch gekennzeichnet, daß die Stücke nach dem Zerschneiden und einer eventuellen Lagerung und nach Auffrischung des Schnitts in der Nähe des Knotens in die Erde gepflanzt werden.

5. Verfahren nach Anspruch 4, dadurch gekennzeichnet, daß der Teil, der dazu bestimmt ist, in die Erde gepflanzt zu werden, zunächst mit pflanzlichen Wurzelbildungshormonen in Berührung gebracht wird.

6. Verfahren nach Anspruch 5, dadurch gekennzeichnet, daß die Wurzelbildungshormone unter den Auxinen und den Zytokininen und ihren Mischungen ausgewählt werden.

7. Verfahren nach einem der Ansprüche 1 bis 5, dadurch gekennzeichnet, daß man das Rindengewebe longitudinal von dem Teil entfernt, der bestimmt ist, in die Erde gepflanzt zu werden, um eine Wurzelbildung an der Oberfläche zu begünstigen.

8. Verfahren nach einem der Ansprüche 1 bis 7, dadurch gekennzeichnet, daß man auf dem Teil, der bestimmt ist, in die Erde gepflanzt zu werden, die Knospen oder die an der Holzoberfläche ruhenden Meristeme entfernt.

9. Verfahren nach einem der Ansprüche 1 bis 7, dadurch gekennzeichnet, daß die Pflanze nach dem Einpflanzen in Erde mit elnem Produkt behandelt wird, das Austreiben von Wurzelschößlingen verhindert.

10. Verfahren nach einem der Ansprüche 1 bis 9, dadurch gekennzeichnet, daß man nach dem Wurzelschlagen elne Veredelung der Stockstücke mit einer anderen Begonienart durchführt.

**Claims**

1. A method for the multiplication and culture of bamboo-shaped species and woody species, the hybrids thereof and the mutants thereof, of the botanical genus Begonia, which consists in taking, as the material for multiplication, pieces of lignified and mature canes, having axillary buds.

2. The method as claimed in claim 1, wherein, on lignification and maturation, the canes are cut, defoliated and sectioned into pieces of which the length corresponds to the desired plant size.

3. The method as claimed in claim 2, in which the initial cutting of canes is carried out between two nodes.

4. The method as claimed in one of claims 1 to 3, in which the canes are planted after sectioning and, if required, storage, after trimming of the cut in the vicinity of the node.

5. The method as claimed in claim 4, in which the part intended to be buried is previously brought into contact with rooting plant hormones.

6. The method as claimed in claim 5, in which the rooting hormones are chosen from auxins and cytokinins and their mixtures.

7. The method as claimed in one of claims 1 to 5, in which the cortical tissue is removed longitudinally over the part intended to be buried in order to promote rooting at the surface.

8. The method as claimed in one of claims 1 to 7, in which, over the part intended to be buried, the dormant meristems or buds are excised at the level of the wood.

9. The method as claimed in one of claims 1 to 7, in which, after burying, the plant is treated with a product which prevents suckering.

10. The method as claimed in one of claims 1 to 9, in which, after rooting, a grafting of the cane sections is carried out with another variety of Begoniacea.

FIG_1

FIG_2

FIG_3

FIG.4

2

FIG.5

FIG.6